# EUROPEAN PATENT SPECIFICATION

(11) **EP 1 786 895 B1**
(45) Date of publication and mention of the grant of the patent: **24.03.2010**
(21) Application number: 05776198.3
(22) Date of filing: 19.07.2005
(51) Int. Cl.: C12N 5/00

(54) **TRIDIMENSIONAL SUPPORT FOR CELL CULTURE, CULTURE METHOD MAKING USE OF SUCH A SUPPORT AND STEM-LIKE CELLS OBTAINED THROUGH SUCH A METHOD**
DREIDIMENSIONALE UNTERSTÜTZUNG FÜR EINE ZELLKULTUR, KULTIVIERUNGSVERFAHREN UNTER VERWENDUNG EINER SOLCHEN UNTERSTÜTZUNG UND DURCH EIN SOLCHES VERFAHREN GEWONNENE STAMMÄHNLICHE ZELLEN
SUPPORT TRIDIMENSIONNEL DE CULTURE CELLULAIRE, PROCEDE DE CULTURE FAISANT USAGE D'UN TEL SUPPORT ET CELLULES DE TYPE CELLULES SOUCHES OBTENUES PAR CE PROCEDE

(30) Priority: 27.07.2004 IT RM20040379
(43) Date of publication of application: 23.05.2007
(73) Proprietor: UNIVERSITA' DEGLI STUDI DI ROMA "LA SAPIENZA", 00185 Roma (IT)
(72) Inventor: CALVIERI, Stefano, 00185 Roma (IT); OTERI, Giovanni, 00185 Roma (IT)
(74) Representative: Germinario, Claudio
(86) International application number: PCT/EP2005/053489
(87) International publication number: WO 2006/010727

(56) References cited:
- WO-A-00/47129
- WO-A-96/03094
- WO-A-2004/040206
- WO-A1-99/47644
- US-A- 5 409 829
- PELLEGRINI G ET AL: "The control of epidermal stem cells (holoclones) in the treatment of massive full-thickness burns with autologous keratinocytes cultured on fibrin." TRANSPLANTATION. 27 SEP 1999, vol. 68, no. 6, 27 September 1999 (1999-09-27), pages 868-879, XP008062490 ISSN: 0041-1337 cited in the application
- MATHOR M B ET AL: "Clonal analysis of stably transduced human epidermal stem cells in culture." PROCEEDINGS OF THE NATIONAL ACADEMY OF SCIENCES OF THE UNITED STATES OF AMERICA. 17 SEP 1996, vol. 93, no. 19, 17 September 1996 (1996-09-17), pages 10371-10376, XP002375540 ISSN: 0027-8424 cited in the application
- DUNNWALD M. ET AL: 'isolating a pure population of epidermal stem cells for use in tissue engineering' EXPERIMENTAL DERMATOLOGY vol. 10, no. 1, February 2001, pages 45 - 54

## Description

### Technical field of the invention

The present invention relates to tridimensional cell culture \supports capable of keeping cultivated cells in the stem compartment and/or of fostering their return to an undifferentiated state, in particular supports for keratinocyte culture. The invention further relates to methods for obtaining stem-like cells.

### Prior state of the art

In an adult, many tissues like the stratified epithelia of the cutis, the intestinal epithelium, the gastric mucosa, the mucosa of the respiratory tree and the bone marrow are in a process of constant regeneration. Terminally differentiated cells are continuously destroyed and lost from the surface of tissues and replaced through highly controlled and complex proliferative processes. Several known factors stimulate and regulate cell differentiation and proliferation in the formation of new tissues. Among them, the growth factors, the cytokines, the spatial interactions between cells and various components of the extracellular matrix.

Somatic stem cells underlying these tissue regeneration processes are undifferentiated cells whose presence has been reported among differentiated cells of several tissues, like liver, pancreas, brain, bone marrow, bone, cartilage, muscles, olfactive epithelium, intestinal epithelium and epidermis. While in some kinds of tissues, like the nervous tissue, stem cells seem relatively inactive in the adult, in other tissues like epidermis or intestinal mucosa stem cells maintain a marked activity in the post-natal life as well.

In epidermis, for instance, cell proliferation and differentiation are compartmentalized and finely regulated. *In vivo*, in hairless regions, this process is achieved by two distinct subpopulations of keratinocyte progenitors found at the level of the basal layer: stem cells and transient amplifying cells.

Stem cells are a subpopulation of relatively quiescent cells, yet characterized by a great proliferative potential and a practically endless capacity for self-renewal. Their function is to maintain cell homeostasis; they exhibit reduced mitotic activity and generate daughter cells, the subpopulation of TA cells (Transient amplifying cells), which rapidly replicate forming a cell colony that attains terminal differentiation within 4-5 days. Moreover, in the cutaneous stem system belong the cells of the bulge and of the hair follicle matrix, the cells of the ungual matrix and of the sebaceous, apocrine, eccrine, holocrine glands.

TA keratinocytes, immediately after their exiting the stem compartment, exhibit a great reduction of *p53* expression, though still possessing an appreciable proliferative capacity. The proliferation process is controlled by clonal evolution, i.e. by the continuous transition from stem cells to TA cells. The down-regulation of genes involved in the cell cycle prevents the clonal evolution of the keratinocytes and keeps the same in the stem compartment. All this enables primary human keratinocytes to bypass senescence.

The availability of stem cell populations is fundamental to cell therapy, which is a novel therapeutic strategy aimed at replacing or repairing serious tissue damages with cells in culture. The success of this therapy requires effective methods of cultivation and transplant of the different populations of somatic stem cells. The limit lies in the difficulty, typical for all tissues, of isolating the stem cells from the patient's adult somatic cells. In the specific case of the stem keratinocytes and TA cells, this requires an incubation of the cells in culture with a radioactive substance, tritiated thymidine, that at DNA duplication may be incorporated in the cell nucleus and therefore highlight the cells with a faster replicative cycle (pulse of tritiated thymidine).

WO96/03094 and WO00/47129 disclose the use of semipermeable membranes with topographic features such as grooves or peaks and troughs to fabricate a skin substitue. However unlike the culture support of the present invention, the features of the membranes of WO96/03094 and WO00/47129 are only foreseen to reach a maximum width of 500 microns.

Scope of the present invention is to provide an effective technique allowing the culture and the isolation of stem cells of different histological origin, in particular of the epidermis, which may be conducted simply and inexpensively.

### Summary of the Invention

The present invention is based on the discovery that the maintenance of the stem characteristics of cells of various tissue origin and/or the return to such characteristics by cells already differentiated or partially differentiated is due to an array of signals getting to the cell itself owing to the fact that the latter has its own well-defined location inside the tridimensional structure of the tissue to which it belongs, and the fact of participating in the whole cytoarchitectonic organization in which cell interaction plays an essential controlling role in cell differentiation. For instance, an isolated, differentiated or partially differentiated keratinocyte can assume stem cell characteristics when cultivated under suitable conditions, but above all when placed in the predetermined position inside the epidermal ridge. Hence, aim of the invention is to artificially recreate a culture environment as similar as possible to the natural architectures of the various tissues, among these preferably the cutis.

In light of the above, object of the invention is a cell culture support made with an anelastic deformable semipermeable material configured so as to reproduce the natural tridimensional architecture of the tissue to which the cells in culture belong. Preferably, such a support is a semipermeable membrane comprising depressions, in the form of wells, invaginations or introflections and ridges, reproducing the organization and the natural tridimensional structure of the corresponding tissue, like the epidermal, intestinal, corneal, bone, cartilaginous, nervous tissue. Depressions and ridges reproduce in particular the shape, the dimensions and the tilt with respect to the tissue surface, of the natural structures.

A second object of the invention is a device for cell culture comprising culture chambers separated, yet communicating through the semipermeable culture support of the invention.

A further object of the invention is a method for obtaining stem cells, wherein differentiated cells taken from adult tissues are cultivated on the cell culture support of the invention, the cell clones grown on the bottom of depressions are isolated and further propagated on the same culture support or on other supports. Advantageously, the differentiated cells are adult keratinocytes, the cell clones grown on the bottom of the wells, invaginations or introflections are stem keratinocytes. Keratinocytes are cultivated on a layer of irradiated fibroblasts, physically separated from the latter by the same semipermeable culture support.

Further objects of the invention are various methods of preparation of the culture support and of the culture device comprising the support itself.

The present invention allows to easily and quite inexpensively obtain a population of cells having somatic stem cell characteristics, or at least a part thereof. Right from the initial culture cycles, this population proves highly homogeneous and essentially devoid of contamination from adult differentiated somatic cells.

### Brief description of the figures

Figure 1: the figure schematically illustrates the stratified and tridimensional structure of the cutis. Evident in the bottom portion of the scheme are the dermo-epidermal membrane invaginations, at the bottom of which the stem compartment lies. Therefrom, the stem keratinocytes start their differentiation path toward the more superficial layers of the cutis.

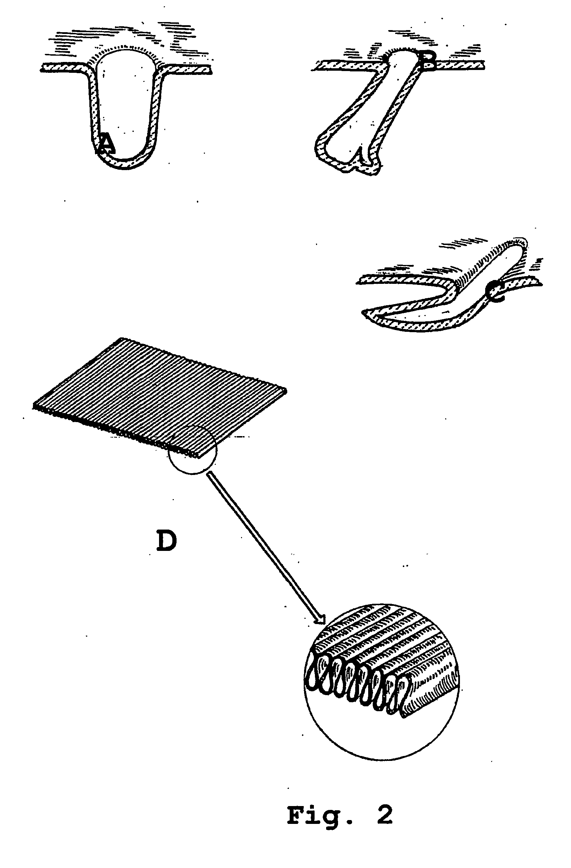

Figure 2: Panel A depicts a depression in the form of a nearly cylindrical well reproducing the invaginations housing the stem compartment of the keratinocytes deputed to formation of the corresponding cutis layers. Panel B depicts the bulb-shaped introflection with an axis tilted with respect to the cutis surface typical of the stem compartment of the hair follicle. Panel C illustrates the markedly tilted laminar introflection housing the stem cells of the ungual matrix and the corresponding annex. Panel D illustrates an alternative embodiment of the invention obtained by folding of a membrane in sac-shaped repeating curls, reproducing the succession of introflections and ridges of the deep layer of tissues. Panel E shows two coaxial cylindrical structures, the internal one thereof consisting of the same semipermeable membrane, separated by a 50-100 µ crown. In this space endothelial cells or nervous cells should be laid.
Figure 3: the figure illustrates the embodiment of a culture support according to the invention. The membrane is shaped with depressions that may have a round or elongate lumen.
Figure 4: Insert^{®} culture device comprising cups having a bottom made with the support of the invention, and cup-containing capsules.
Figure 5: schematic depiction of a culture cup.
Figure 6: Panel A reports the results of the viability analysis of keratinocytes cultivated on plane culture surface. The graph shows how the percent absorption values at 540 nm (y-axis) which are the index of cell viability, quickly decrease over time (X-axis) reaching the baseline after about 40 hours of culture (at +40 h). Panel B reports the results of the same analysis conducted on cells cultivated in the plane zone of the culture support of the invention.
Figure 7: Panel A illustrates the results of the viability analysis of clones obtained from plane-zone keratinocytes at +48 h, or, Panel B, at +6 days.
Figure 8: Panel A reports the results of the viability analysis of clones of "vallecula" keratinocytes, i.e., cultivated on the bottom of depressions. Panel B reports the results of the viability analysis of clones of deep-zone keratinocytes at +4 days.
Figure 9: Panel A: viability of deep-zone keratinocyte clones at +10 days; or, Panel B, of clones obtained from deep-zone keratinocyte lines at +4 days.
Figure 10: Panel A reports the results of LDH release by keratinocytes in culture. Light-colored bars correspond to keratinocytes cultivated in the plane zone; dark-colored bars correspond to deep-zone keratinocytes. Panel B reports the results of Nitrogen oxide release by the keratinocytes in culture. Light-colored bars correspond to the keratinocytes cultivated in the plane zone; dark-colored bars correspond to deep-zone keratinocytes.
Figure 11: Panel A reports the organization of proteins of the cytoskeleton of keratinocytes cultivated in the valleculas, or, Panel B, in the plane zone. Light-colored bars correspond to keratinocytes cultivated in the plane zone; dark-colored bars correspond to deep-zone keratinocytes.

### Detailed description of the Invention

For "culture support" it is meant any material compatible with the growth and the propagation of heterogeneous-phase cells. Cells are seeded on the surface of the culture support and bred maintaining adherence at least for the first propagation cycle.

The culture support according to the invention is a semipermeable anelastic material, deformable under pressure without loss of semipermeable properties. Commercially available synthetic or natural semipermeable membranes of the kind commonly employed in cell culture may be used as starting product; e.g., polyethylene terephtalate (Tradename PET). Other materials, like assemblies or cartridges of identical or different semipermeable membranes, or microporous polymer materials, may likewise be used when their chemical nature is not incompatible with the adhesion and the growth of cell cultures.

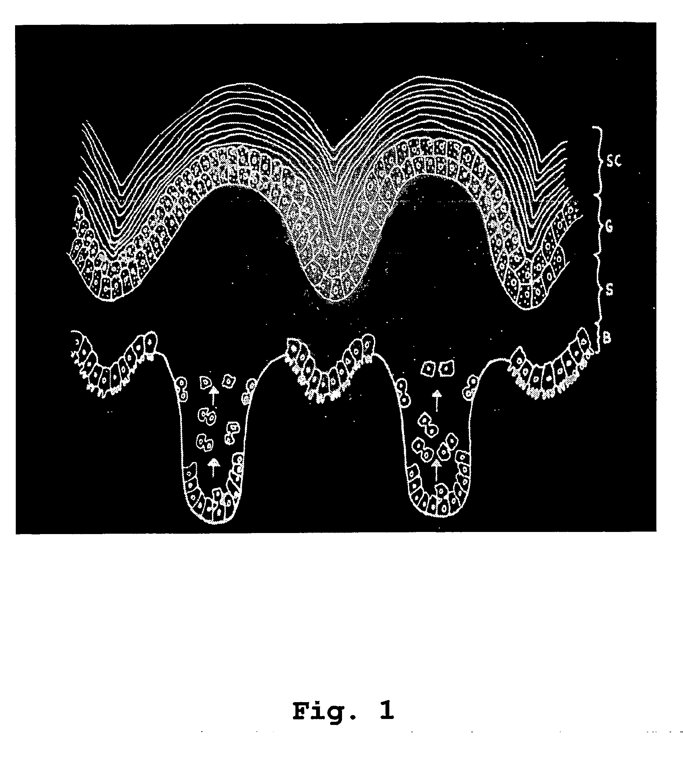

Already the mere semipermeable characteristic of the support carries out several relevant functions; the first one is that of reproducing as best one can the shape of the natural support of adhesion and growth. In fact, it is known that stem cells of epithelial tissue adhere and grow on a semipermeable lamina separating them from the underlying connective tissue. E.g., keratinocytes lie and are adhered onto the dermo-epidermal junction, a structure that can be likened to a semipermeable membrane and that separates them from the underlying derm rich in fibers and fibroblasts (Figure 1). A second function is that of allowing the free diffusion of the culture medium and of the nourishment.

A third relevant function has specifically been singled out in connection with keratinocyte cultures. It is known, from data in literature, (Mathor Monica B. et al. Proc. Nat. Acad. Sci. USA Vol. 93, pp. 10371-10376, September 1996) that cultures of these cells remain viable when seeded on a layer of lethally irradiated (3T3) feeder fibroblasts. Clearly, the presence of a second cell type not only can influence in a non-reproducible manner the results obtained, but evidently it hinders the attainment of a homogeneous and pure population of stem clones, requiring a subsequent complicated process of selecting and separating the different cell populations. Also this specific problem is solved by the invention. In fact, it has surprisingly been observed that the stimulation effect exerted by the layer of 3T3 feeder fibroblasts on the keratinocyte cultures does not depend on the direct contact of the different cell populations, but merely on the sharing of the same culture medium. Therefore, the invention offers the futher advantage of allowing the culture of keratinocytes in a form physically separated from the layer of feeder fibroblasts thanks to the semipermeable support, forming a barrier between the two populations without however hindering the required diffusion of the culture medium and of the factors produced by the fibroblasts.

Evidently, this advantageous option is applicable to the production of homogeneous populations of stem cells of different origin, any time cell growth has to be supported by the co-presence of feeder cells.

The semi-permeable characteristics of the material of the invention are not *per se* sufficient to reproduce the natural environment of growth and development of the cells in culture. The capacity of the material to deform and the inelasticity are fundamental properties so that the material may be suitably shaped with depressions and/or ridges, and may maintain the received shape. Also these characteristics are met by the above-considered commercially available membranes.

By exerting suitable stresses, pressure or other manipulations on such membranes it is possible to cause, with respect to the membrane plane, invaginations or introflections, also called "valleculas", that, evenly repeating on the extension of the entire membrane, reproduce the tridimensional architecture with plane zones and depressions of the desired tissues.

The shape and the tilt of such depressions with respect to the membrane plane exert an essential role in addressing the differentiation of stem cells to the desired tissue or tissue annex. For instance, the epithelial tissues of the cutis comprise stem cells deputed to production not merely of keratinocytes, but also of cutaneous annexes like hair, head hair, nail, sebaceous, apocrine, eccrine, holocrine and mammary gland. The stem district deputed to keratinocyte production lies on the bottom of near-cylindrical introflections, orthogonal to the cutis surface, created by the semipermeable lamina and reproduced on the support of the invention as illustrated in figure 2A. The stem district deputed to regeneration of hair annexes, hair and head hair, lies in the deep terminal region of introflections shaped like an elongate bulb having circular lumen and major axis tilted of about 30° to 45° with respect to the cutis surface and reproduced on the support of the invention as illustrated in figure 2B, whereas the district deputed to generation and growth of ungual annexes lies on the bottom of laminar epidermal introflections, having elongate lumen and a major axis nearly parallel to the cutis surface, which are reproduced on the support of the invention as illustrated in figure 2C.

Introflections of desired contour, dimensions and tilt are produced by causing the superficial deformation of the material, as well as by folding of a membrane into sac-shaped curls, repeating and blocked in the desired shape by suitable securing means, like a framework or other rigid supporting structure (Figure 2D).

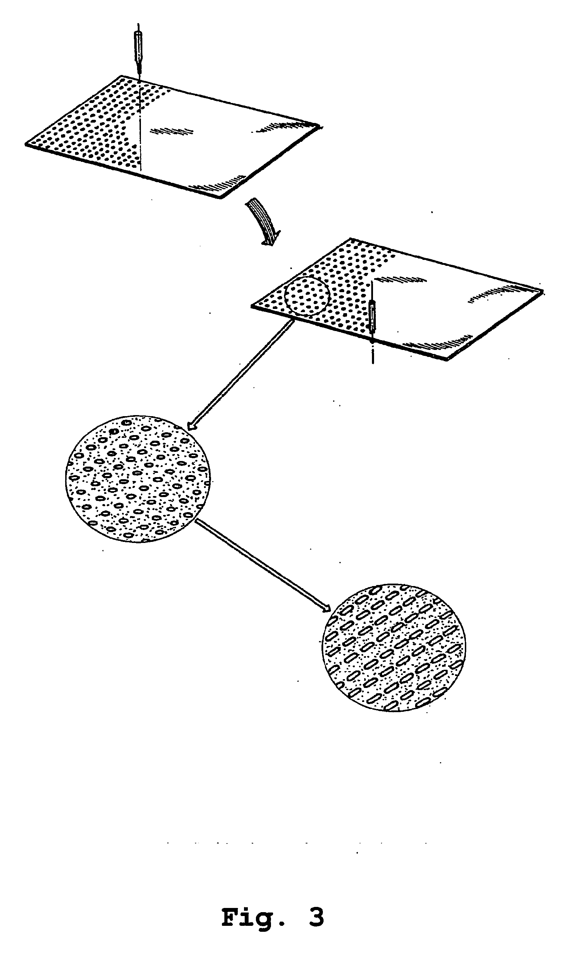

In the preferred embodiment, the material is a membrane in which even-spaced depressions are obtained with one or more punches, or equivalent instrument, having rounded point and a shape complementary to the invagination contour (Figure 3). The tilt of the punch with respect to the membrane surface determines the tilt of the invagination. Such a tilt is subordinated to the tissue architecture that is desirable to reproduce, and it is of, e.g., about 90° for even wells, or from 30° to 45° for wells suitable for hair annexes, or from 5° to 35° for ungual annexes. Automatic systems making use of sets of punches capable of shaping large surfaces of material or membrane in a single pass can of course be used at industrial level. Moreover, depressions of desired shape and dimensions can be produced by subjecting a membrane, suitably positioned on a rigid and shaped mould, to a sufficiently high pressure or vacuum, in order to deform it, forcing it to invaginate in the depressions of the mould. Any other alternative method attaining the same effect, such as the direct on-mould synthesis of membranes, may be used as well.

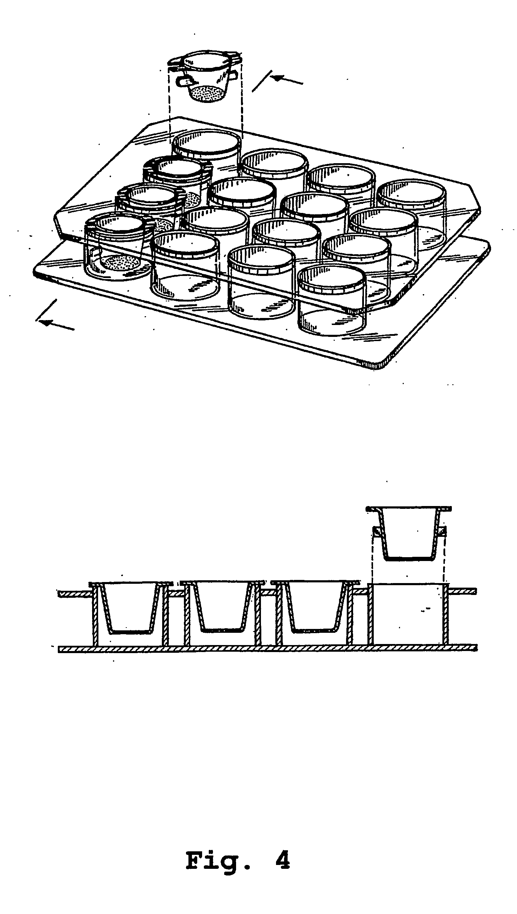

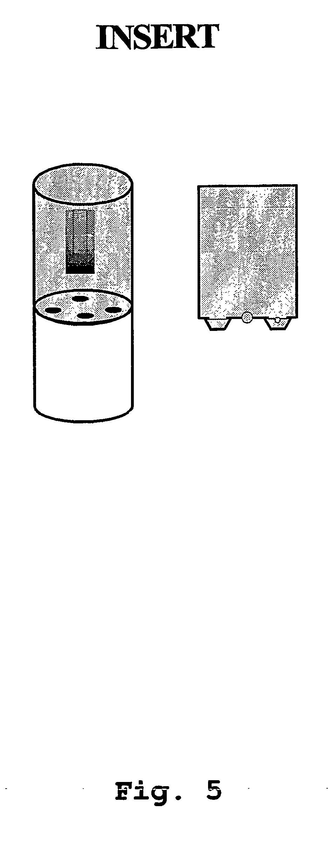

The dimensions of the depressions should be consistent with the real dimensions of the tissue indentations. However, larger dimensions are desirable, when not compromising the production of the desired stem cells, as yielding heftier cell masses, with an obvious advantage at the industrial level. Usually, the dimensions of the lumen of the depressions range from a few tens of microns, e.g., 30 or 50 µm, to hundreds of microns, e.g., 200, 400, 600, 900, 1000 or 2000 µm, preferably about 1000 µm. The lumen may be roughly round, oblong or markedly elongate when the depression is a furrow. Also the depth of the depressions will be consistent with the cultivated cell type. It is known, e.g., that bulb-shaped depressions containing the hair follicle introflex until reaching the deeper layers of the cutis, whereas furrows containing the ungual matrix develop in a zone relatively superficial and basically parallel to the cutis surface. The depth will range from 50 to 2000 microns, preferably from 100 to 1000 microns, e.g., it will be of 200, 500, 1000, 1500 µm. The culture support of the invention is incorporated in a rigid device for cell culture, facilitating the use thereof. Such a device may comprise one, two or more culture chambers separated by the semipermeable material or membrane according to the invention. The system with more chambers allows to cultivate the desired cells, in a medium shared by other cell cultures, however keeping the different cell populations physically separated. This is the case of keratinocytes, remaining viable only when co-cultivated with feeder cells, i.e., irradiated fibroblasts. In the preferred embodiment of the invention, the device is a variant of the Insert^{®} system marketed by Falcon Industries, comprising a plane support plate in which one or more wells or capsules are opened, and a set of cups located within such capsules. The cups have their bottom made by the semipermeable culture support of the invention, be it a mere membrane or a rigid cartridge obtained by membrane folding as described above. Moreover, the dimensions of the cups are such that, once located within the capsule, the cup bottom is spaced apart from the capsule bottom. This allows the culture of a first cell population on the cup bottom, i.e. the support of the invention, and of a second cell population on the capsule bottom, without those populations coming into physical contact therebetween, though sharing the same culture medium. Of course, any equivalent variant of this system may likewise be used (Figures 4 and 5).

The cell culture method according to the invention provides that cells obtained from adult tissue samples containing cells in various differentiation stages be seeded on the entire surface of the support and cultivated using methods and operative conditions well-known to a person skilled in the art. In the case of keratinocytes, these are obtained from explants of healthy dissociated and frozen cutis containing keratinocytes in various differentiation stages. Cells seeded in the plane zone or in the support introflections originate two distinct cell populations having different behaviors. In particular, cells cultivated on the bottom of the introflections exhibit characteristics typical of somatic stem cells. Not only are the former capable of forming colonies that maintain a high mitotic index and a prolonged viability over time; they are also capable of retaining the cell cycle control mechanisms, like the G2/M phase, which prevent the clonal evolution of the cells, keeping them in the stem compartment, and leading the cell itself to death by apoptosis and not by necrosis/inflammation, as in the case of conventionally cultivated adult cells.

Hence, the experimental data reported below demonstrate that differentiated or partially differentiated cells obtained from sampling of adult tissues, cultivated in the tissue structure regions deputed to production and maintenance of stem cells, regress to the undifferentiated state typical of somatic stem cells.

Hence, cells collected from the deepest region of the introflections can be serially propagated on the same support or on other support, and generate populations of stem-like cells highly homogeneous and essentially devoid of adult different cells. Lastly, cell lines can be obtained *in vitro* by immortalizing cell clones isolated from said populations following techniques and conditions well-known to a person skilled in the art. Adult cells useful as starting product in the method of the invention are those obtained from biopsies of intestinal epithelium, respiratory epithelium, cutis, olfactory epithelium, cornea, liver, pancreas, nervous tissue, bone marrow, bone tissue, cartilage, muscle tissue.

The invention, by allowing the production of large amounts of somatic stem cells, offers ample options of development to cell therapy, which is aimed at replacing or repairing serious tissue damages through stem cell cultivation and transplant. For instance, epidermis regeneration obtained with cultivated autologous keratinocytes can be decisive for patients suffering from massive third-degree burns. Grafts of cultivated epidermis can be used, under local anesthesia and without scarring, for the treatment of large body areas of patients affected by stable vitiligo. In addition, limbal stem cell cultivation and transplant reconstructs the corneal surface and restores visual acuity to patients with serious ocular surface damages due to total defectiveness of limbal stem cells. Lastly, the use of somatic epidermal stem cells in genodermatoses (like, e.g., in ichthyoses or in epidermolysis bullosa) should not be underestimated.

### Experimental examples

### Keratinocyte culture method

Keratinocytes obtained by sampling of healthy donors were cultured according to the protocol of Mathor et al., 1996 (supra) on the bottom of Insert® cups (Falcon Industries) (figure 4) made with the culture support of the invention. 3T3 Cells, of fibroblast nature, irradiated (Mathor et al. 1996) and coming from the Tissue Bank of the Istituto Superiore di Sanità (Italy), are cultivated on the bottom of the capsule of the same Insert® device, maintaining the physical separation between keratinocytes and fibroblasts. However, cups and capsules are immersed in the same culture medium. For serial growth, the cells were passed to the subconfluence stage to prevent their senescence in accordance with the method described by Pellegrini et al. "Transplantation", Vol. 68(6), pp 868-879, 27 September 1999.

### Method for determination of viability and colony-forming efficiency.

To calculate cell viability, Neutral Red assay was used. Neutral Red is a vital dye entering lysosomes of viable cells. The amount of dye extracted from the cells is read at the spectrophotometer and is proportional to the number of viable cells. The colony-forming efficiency was obtained by plating the cells at clonal density and leaving them to multiply in culture for 7 days. Then, they were stained with Neutral Red and counted at the spectrophotometer, as in the investigation of viability.

### Immunohistochemistry and immunofluorescence

The cells were fixed with water-diluted 3.7% paraformaldehyde and incubated 15 min at room temperature. Three washings with PBS saline and permeabilization with acetone for 5 min at 20°C. Upon sucking off the acetone, cells were left drying in air for 5 min. Then, two different kinds of antibodies were used: rhodamine-conjugated phalloidine for actin labeling and a monoclonal antibody for tubuline labeling. Phalloidine is a mushroom (*Amanita Phalloides*)-extracted toxin, binding irreversibly to actin molecules. For nuclear DNA labeling a fluorescent dye was used; 4',6-Diamidino-2'-phenylindole (DAPI), specifically binding to DNA and forming markedly fluorescent complexes having high specificity therewith.

### Method for determination of lactic dehydrogenase (LDH) release

To investigate LDH release, it was carried out a specific assay using a kit basing the detection of the enzyme release in the culture medium on the reduction of NAD to NADH in the presence of LDH and on the subsequent conversion of tetrazolium to formazan, an insoluble salt whose formation can be read at the spectrophotometer.

### Method for determination of Nitrogen oxide release

The cells in culture were prepared for the assay with Griess reagent, which binds to the produced nitrite, forming a red-stained compound that is read at the spectrophotometer.

### Results

Starting from the experimental model of the invention, there were obtained two cell populations exhibiting different characteristics: cells in culture on the plane zone of the support, and cell in culture on the bottom of the introflections.

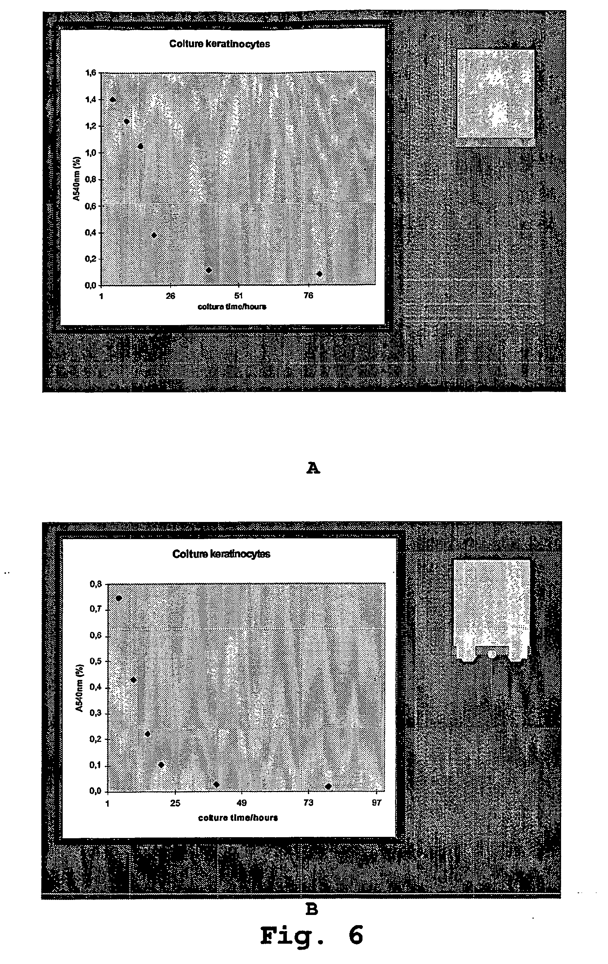

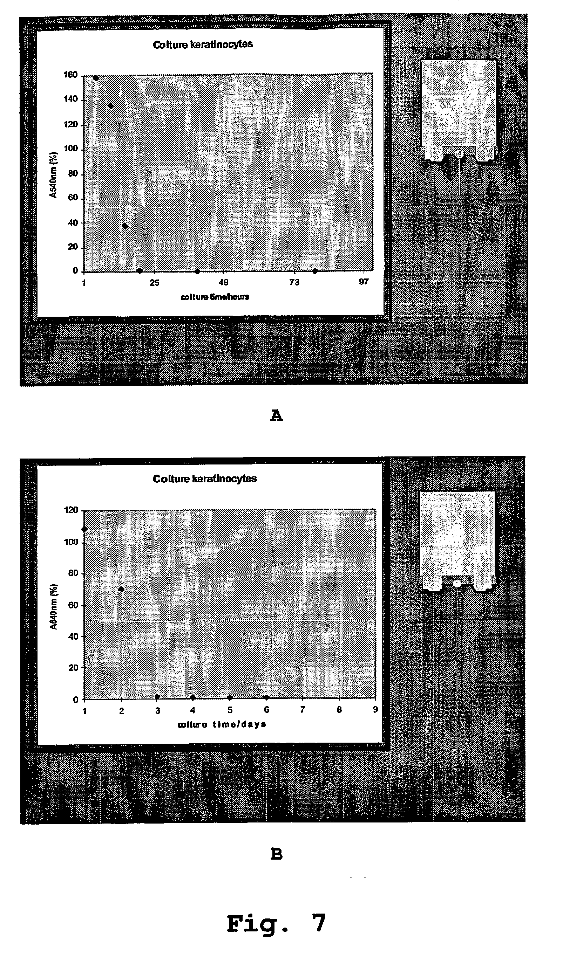

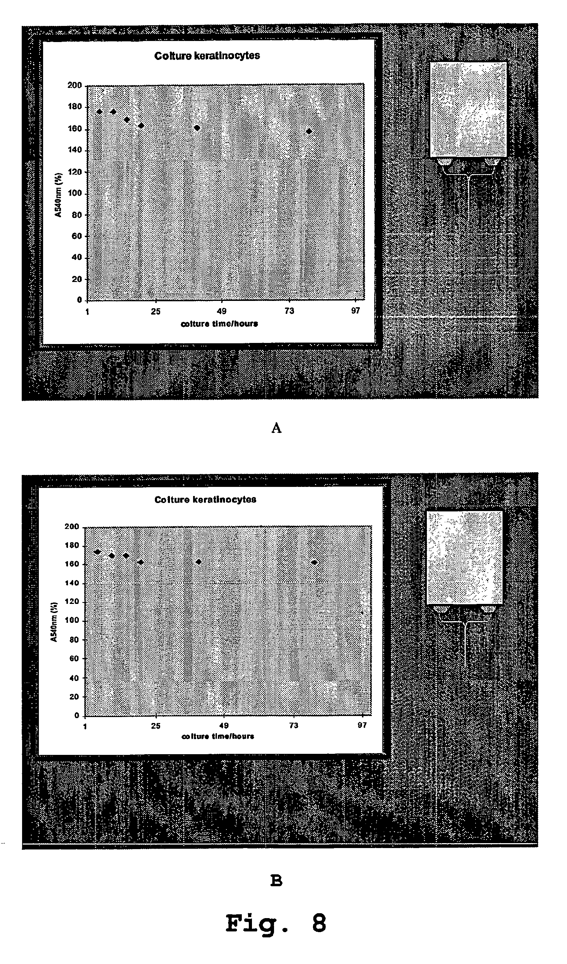

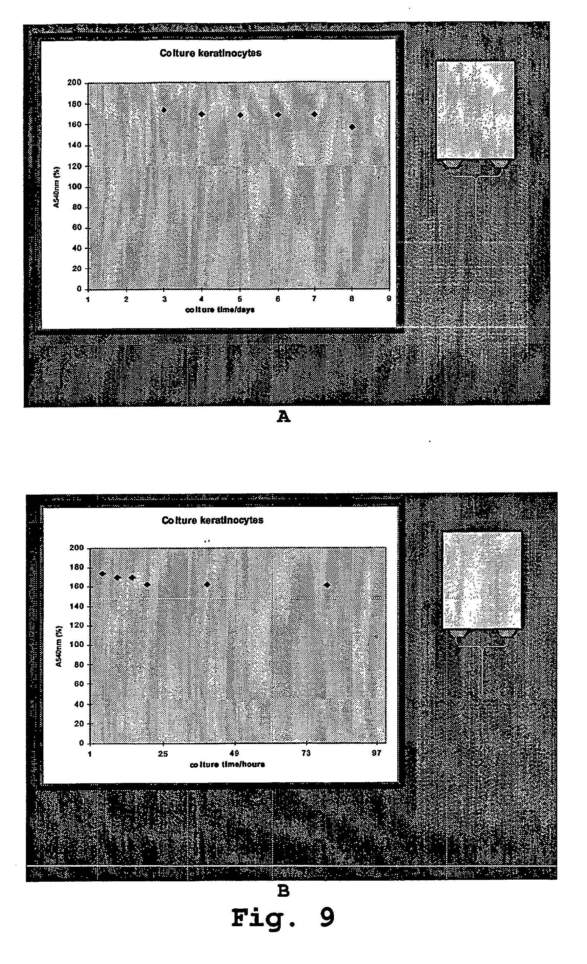

These two distinct populations exhibit different capacities of keeping viable for more days in culture and different colony-forming capacities. The viability profile of clones obtained from keratinocytes cultivated on a plane surface or in the plane zone of the support of the invention shows a fast fall of the absorption values at 540 nm (A540 nm %) and therefore of the viability with death of all clones within the first 3 days (figures 6A, 6B, 7A and 7B). On the contrary, the viability profile of clones obtained from keratinocytes cultivated on the bottom of depressions of the support of the invention shows maximum and constant absorption values for over 10 days, something that highlights high viability values of the corresponding clones (figures 8A, 8B, 9A and 9B).

This latter characteristic is to date one of the universally recognized properties of stem cells. Aside from the viability and colony-forming capacity studies, cell labelings have been conducted with immunofluorescence technique to check changes in the cytoskeleton of the cells in culture. In particular, there have been investigated modifications in the actin and in the tubulin, which is more involved in cell cycle events.

Normal cells owe their shape to a tridimensional framework of filaments formed by polymerization of the actin protein.

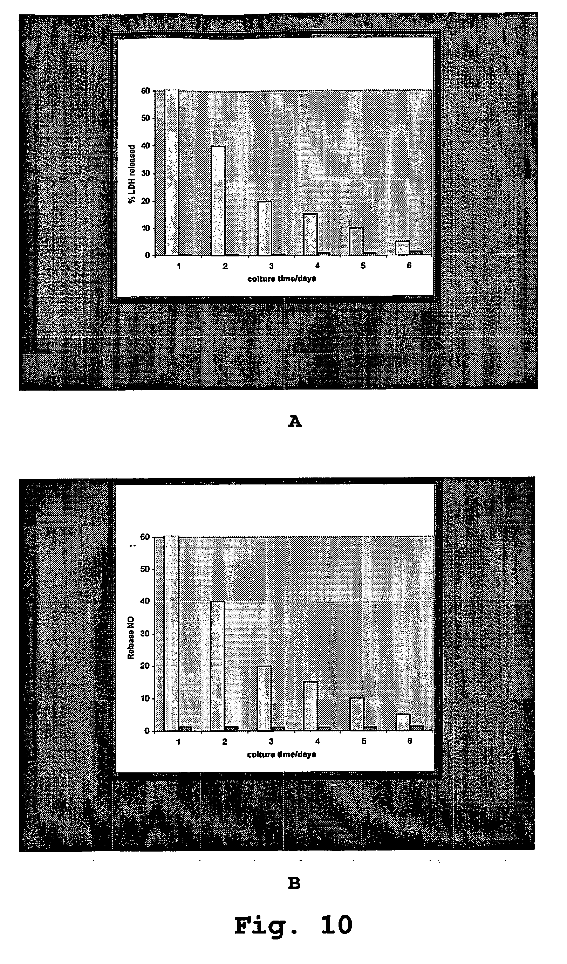

Only half of the actin contained in a cell is polymerized in microfilaments, the remainder being in the form of soluble globular actin. The polymerization-depolymerization of the filaments of this protein is a rapid process occurring in both directions, regulated by specific molecules through a mechanism that is only partially known. In normal quiescent cells, polymerized actin is organized into bundles of various dimensions formed by parallel fibers joined by a set of accessory proteins. Under physiological conditions, actin fibers reversibly disassemble when the cell divides, contracts or, somehow, moves. This organization of the cytoskeleton proteins is evident in cells of colonies obtained from vallecula keratinocytes (Figure 10A), whereas in cells obtained from plane-zone colonies the presence of actin in bundles of organized fibers is exceptional (Figure 10B). The actin is for the most part diffused in soluble monomer form, or thickened in disordered masses, mostly localized in the peripheral portion of the cell, near the membrane area making contact with the culture support. These structures are quite similar to those described in tumor cells, known as "podosomes". In normal cells, the ends of the actin bundles are anchored to specific plasma membrane structures, called adhesion plates. The anchoring is provided by other proteins, firmly joining actin to a complex of molecules called integrins. Integrins are receptors of electric charges of the extracellular environment and of extracellular matrix proteins: they are proteins themselves, localized in the adhesion plates, crossing the plasma membrane from side to side. Integrins are a very wide family of receptor molecules; some receptors are single-chain, others are double-chain, being composed of two subunits called alpha and beta. Phosphorylations induced in the integrins are accompanied by modifications of the cytoskeleton and of the adhesion plates. In normal cells, the disorganization of the adhesion plate structure and the likely consequent cytoskeleton disanchoring is capable of activating a cascade of second messengers, leading, as last step, to the activation of apoptosis-inducing genes. Then, all changes in the nucleus during the step of culturing were checked in the various cell types. In order to do this, there were used immunofluorescence techniques with a fluorescent dye, the DAPI (above), binding specifically to DNA and forming therewith markedly fluorescent complexes having high specificity. Such labelings highlighted that the death of the colonies obtained from support introflections after 9 days of culture occurs by an apoptotic mechanism highlighted by the specific fragmentation of the nucleus and the formation of the characteristic blebs, whereas no nuclei at a necrotic-inflammatory stage where found. On the contrary, the colonies obtained from plane zone cells die by a necrotic-inflammatory mechanism. In order to confirm the kind of death incurred by the different cell types, releases of both LDH and nitric oxide by the different populations were determined. In particular, lactic dehydrogenase release is typical of cell membranes of cells dying with a necrotic process, whereas in apoptotic cells the membrane tends to stiffen with an entailed reduced leak of the cytoplasmatic enzyme.

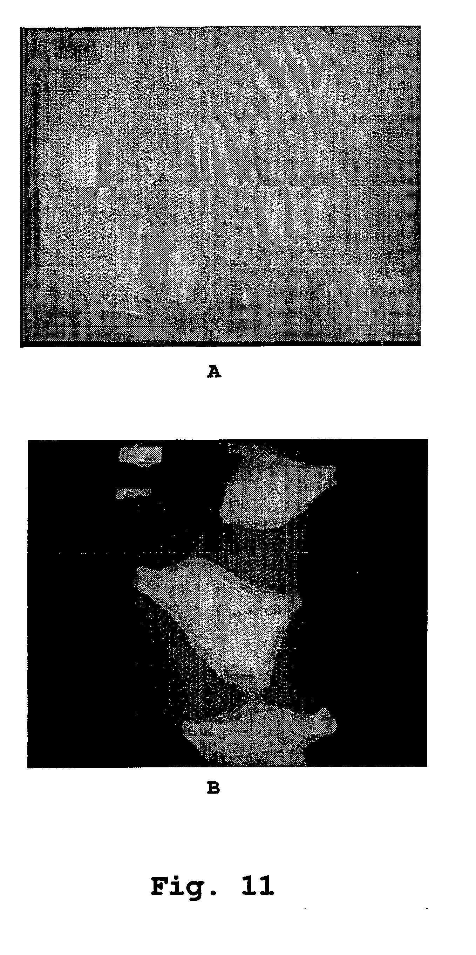

The measuring of Nitrogen oxide release indicates a possible necrotic/inflammatory process as cause of cell death. For the study of LDH release a kit was used based on NAD reduction to NADH in the presence of LDH and on the subsequent conversion of tetrazolium to formazan, an insoluble salt whose formation can be read at the spectrophotometer. From the graphs in figure 11A it may be seen how only the plane-zone cells release LDH in the medium, unlike the negligible levels of enzyme released by the deep-zone keratinocytes. Likewise, Nitrogen oxide release was studied by means of an assay using Griess reagent, which binds to the produced nitrite forming a red-stained compound that is read at the spectrophotometer. In this case as well, the graph in figure 11B highlights that only plane zone keratinocytes release Nitrigen oxide in the colture medium, unlike deep zone cells. All this data confirm the hypothesis that death of the plane zone cells, as well as that of the keratinocytes in culture according to the previously known methods, occurs with a necrotic-inflammatory process, whereas death of the deep zone keratinocytes occurs by an apoptotic process. This indicates that the deep zone keratinocytes are not only capable of forming colonies maintaining a high mitotic index and a viability protracted over time, but also of maintaining the cell cycle control mechanisms preventing the clonal evolution of the keratinocytes and keeping them in the stem compartment, leading the cell itself to death by apoptosis and not by necrosis-inflammation.

## Claims

1. A cell culture support, **characterized by** being an anelastic deformable semipermeable membrane comprising depressions and ridges of shape and tilt configured so as to reproduce the natural tridimensional architecture of the tissue to which the cells in culture belong, wherein the depression have depth of more than 500 to 1000 microns and a lumen of more than 500 to 2000 microns.

2. The culture support according to claim 1, **characterized in that** the depressions are wells or invaginations with circular or oblong lumen, or furrows.

3. The culture support according to claim 1, **characterized in that** the depressions and the ridges are produced by a set of subsequent introflections, originating blind-bottom sac-shaped formations packed in a rigid structure.

4. The culture support according to any one of the claims 1 to 3, **characterized in that** it reproduces the tridimensional architecture of the epidermal, intestinal, corneal, bone, cartilagineous, nervous tissue.

5. The culture support according to claim 4, **characterized in that** it reproduces the succession of dermal ridges and epidermal invaginations typical of the cutis and of the cutaneous annexes.

6. The culture support according to claim 5, **characterized in that** the invaginations have a tilt with respect to the membrane surface of about 90° or from 30° to 45° for wells suitable for hair annexes and from 5° to 35° for wells suitable for ungual annexes.

7. A method of preparation of the support according to any one of the claims 1 to 6, **characterized in that** an anelastic, deformable, semipermeable material is subjected to punching with punches of suitable dimensions, or it is folded in a succession of introflections and packed in a rigid framework.

8. A device for cell culture, **characterized in that** it comprises culture chambers separated, yet communicating through the semipermeable culture support according to any one of the claims 1 to 6.

9. The device according to claim 8, comprising a plane support plate in which one or more wells are opened, and one or more cups having a bottom made by the semipermeable culture support and of dimensions such that, when located inside the respective wells, said support be spaced apart from the bottom surface of the well.

10. A method for obtaining stem cells, **characterized in that** somatic differentiated cells taken from adult tissues are cultivated on the cell culture support according to any one of the claims 1 to 6, the cell clones grown on the bottom of wells or invaginations are isolated and optionally further propagated to produce populations of stem cells or individual stem cell lines.

11. The method for obtaining somatic stem cells according to claim 10, wherein the cultured cells are keratinocytes from adult tissues, the cells clones grown on the bottom of the wells or invaginations are stem keratinocytes and wherein the keratinocytes are cultivated on a layer of irradiated fibroblasts, physically separated from the latter by the culture support.

## Patentansprüche

1. Zellkulturträger, **dadurch gekennzeichnet, dass** er eine nicht-elastische deformierbare semipermeable Membran ist, die Vertiefungen und Erhöhungen mit einer Gestalt und Neigung umfasst, die so ausgestaltet sind, dass sie die natürliche dreidimensionale Architektur des Gewebes reproduzieren, zu dem die in Kultur befindlichen Zellen gehören, wobei die Vertiefungen eine Tiefe von mehr als 500 bis 1000 Mikrons und ein Lumen von mehr als 500 bis 2000 Mikrons haben.

2. Kulturträger nach Anspruch 1, **dadurch gekennzeichnet, dass** die Vertiefungen Mulden oder Einstülpungen mit kreisförmigem oder länglichem Lumen oder Furchen sind.

3. Kulturträger nach Anspruch 1, **dadurch gekennzeichnet, dass** die Vertiefungen und Erhöhungen durch eine Reihe aufeinanderfolgender Introflexionen erzeugt werden, die sackförmige Formationen mit Blindboden hervorbringen, die in eine starre Struktur gepackt sind.

4. Kulturträger nach einem der Ansprüche 1 bis 3, **dadurch gekennzeichnet, dass** er die dreidimensionale Architektur von epidermalem, intestinalem, Hornhaut-, Knochen-, Knorpel- oder Nervengewebe reproduziert.

5. Kulturträger nach Anspruch 4, **dadurch gekennzeichnet, dass** er die Aufeinanderfolge dermaler Erhöhungen und epidermaler Einstülpungen reproduziert, die für die Haut und die Hautanhangsgebilde typisch ist.

6. Kulturträger nach Anspruch 5, **dadurch gekennzeichnet, dass** die Einstülpungen eine Neigung im Bezug zur Membranoberfläche von etwa 90° oder von 30° bis 45° für Mulden haben, die für Haaranhangsgebilde geeignet sind, und von 5° bis 35° für Mulden, die für Nägelanhangsgebilde geeignet sind.

7. Verfahren zur Herstellung des Trägers nach einem der Ansprüche 1 bis 6, **dadurch gekennzeichnet, dass** ein nicht-elastisches deformierbares semipermeables Material mit Stanzen geeigneter Abmessungen gestanzt wird, oder es in einer Aufeinanderfolge von Introflexionen gefaltet und in ein starres Gerüst gepackt wird.

8. Vorrichtung für Zellkultur, **dadurch gekennzeichnet, dass** sie getrennte, jedoch durch den semipermeablen Kulturträger nach einem der Ansprüche 1 bis 6 kommunizierende Kulturkammern umfasst.

9. Vorrichtung nach Anspruch 8, umfassend eine plane Trägerplatte, in der eine oder mehrere Mulden geöffnet sind, und einen oder mehrere Becher, die einen Boden haben, der durch den semipermeablen Kulturträger gebildet wird und Abmessungen aufweist, so dass der Träger von der Bodenoberfläche der Mulde einen Abstand hat, wenn er im Inneren der jeweiligen Mulden befindlich ist.

10. Verfahren zum Erhalten von Stammzellen, **dadurch gekennzeichnet, dass** somatische differenzierte Zellen, die adulten Geweben entnommen sind, auf dem Zellkulturträger nach einem der Ansprüche 1 bis 6 gezüchtet werden, die am Boden der Mulden oder Einstülpungen gewachsenen Zellklone isoliert und gegebenenfalls weiter vermehrt werden, um Populationen von Stammzellen oder individuellen Stammzelllinien zu erzeugen.

11. Verfahren zum Erhalten somatischer Stammzellen nach Anspruch 10, wobei die gezüchteten Zellen Keratinocyten aus adulten Geweben sind, die am Boden der Mulden oder Einstülpungen gewachsenen Zellklone Stamm-Keratinocyten sind und wobei die Keratinocyten auf einer Schicht bestrahlter Fibroblasten gezüchtet werden, die von den letzteren durch den Kulturträger physisch getrennt sind.

## Revendications

1. Support de culture cellulaire, **caractérisé en ce qu'**il s'agit d'une membrane semiperméable déformable anélastique comprenant des creux et des crêtes de forme et inclinaison configurées de manière à reproduire l'architecture tridimensionnelle naturelle du tissu auquel appartiennent les cellules en culture, où les creux ont une profondeur supérieure à 500 à 1000 µm et une lumière de plus de 500 à 2000 µm.

2. Support de culture selon la revendication 1, **caractérisé en ce que** les creux sont des puits ou des invaginations avec une lumière circulaire ou oblongue, ou des sillons.

3. Support de culture selon la revendication 1, **caractérisé en ce que** les creux et les crêtes sont produits par une série de recourbements en dedans successifs, donnant naissance à des formations en forme de sac à fond fermé disposées dans une structure rigide.

4. Support de culture selon l'une quelconque des revendications 1 à 3, **caractérisé en ce qu'**il reproduit l'architecture tridimensionnelle du tissu épidermique, intestinal, cornéen, osseux, cartilagineux, nerveux.

5. Support de culture selon la revendication 4, **caractérisé en ce qu'**il reproduit la succession de papilles dermiques et d'invaginations épidermiques typiques de la cutis et des annexes cutanées.

6. Support de culture selon la revendication 5, **caractérisé en ce que** les invaginations ont une inclinaison par rapport à la surface de la membrane d'environ 90° ou de 30° à 45° pour des puits appropriés pour des annexes capillaires et de 5° à 35° pour des puits appropriés pour des annexes unguéales.

7. Procédé de préparation du support selon l'une quelconque des revendications 1 à 6, **caractérisé en ce qu'**un matériau semiperméable, déformable, anélastique est soumis à un poinçonnage avec des poinçons de dimensions appropriées, ou est plié dans une succession de recourbements en dedans et disposé dans un cadre rigide.

8. Dispositif pour culture cellulaire, **caractérisé en ce qu'**il comprend des chambres de culture séparées, mais communiquant par le biais du support de culture semiperméable selon l'une quelconque des revendications 1 à 6.

9. Dispositif selon la revendication 8, comprenant une plaque de support plane où un ou plusieurs puits sont ouverts, et une ou plusieurs cupules ayant un fond constitué par le support de culture semiperméable et de dimensions telles que, quand elles sont situées dans les puits respectifs, ledit support est séparé de la surface inférieure du puits.

10. Procédé pour obtenir des cellules souches, **caractérisé en ce que** des cellules différenciées somatiques prélevées sur des tissus adultes sont cultivées sur le support de culture cellulaire selon l'une quelconque des revendications 1 à 6, les clones cellulaires qui ont crû sur le fond de puits ou d'invaginations sont isolés et éventuellement propagés encore pour produire des populations de cellules souches ou des lignées de cellules souches individuelles.

11. Procédé pour obtenir des cellules souches somatiques selon la revendication 10 où les cellules cultivées sont des kératinocytes provenant de tissus adultes, les clones cellulaires qui ont crû sur le fond des puits ou invaginations sont des kératinocytes souches et où les kératinocytes sont cultivés sur une couche de fibroblastes irradiés, physiquement séparés de celle-ci par le support de culture.
